# EUROPEAN PATENT APPLICATION

(11) **EP 1 097 682 A2**
(43) Date of publication of application: **09.05.2001**
(21) Application number: 00309567.6
(22) Date of filing: 30.10.2000
(51) Int. Cl.: A61F 13/02

(54) **Wound dressing**

(30) Priority: 05.11.1999 GB 9926252
(71) Applicant: LOHMANN GmbH & Co. KG, 56504 Neuwied (DE)
(72) Inventor: Basedow, Arno Max, 61118 Bad Vilbel (DE); Carus, Edmund Hugh, Clitheroe, Lancashire BB7 9QJ. (GB)
(74) Representative: Barker, Rosemary Anne

(57) **Abstract**

Wound dressing (1) incorporates a rubefacient or hyperemic substance (3) in such a disposition that, upon application of the dressing to a wound site (7) said substance (3) will be brought into contact with skin surrounding the wound site but not with the wound site itself. In this respect, central section (2) of the dressing (1) may be cut away to expose i.e. overlie the wound site. A different type of dressing may be inserted here. The layer of the rubefacient or hyperemic substance (3) may be sandwiched between a cover material (4) and an adhesive layer (5), or it may be incorporated into the adhesive layer or microencapsulated and dispersed through the adhesive layer.

## Description

For many years the presence of warmth at wounds has been known to have beneficial effects in the healing of these wounds. It is well known and documented that raising tissue temperature with applied heat causes dilation of arterial blood vessels which pervade wounds, which in turn results in increased oxygen delivery to these wounds and thus repairing the tissues therein. In particular, the presence of controlled heat, (preferably around 5 degrees Celsius above body core temperature), seems to enhance the quality and rate of wound healing in various wound types ranging from partial thickness types to full thickness wounds in either a clean or infected state.

However, heat therapy for the treatment of wounds, either infected or clean, is extremely difficult to achieve in practice. Devices of various forms have been used but these can result in wound drying or dessication and consequent retardation of the healing mechanisms. Burning of wound sites can also occur.

Efforts have been made over the years to provide devices to control more closely the necessary elevated temperatures required for optimum wound healing. French Patent 1 527 887 (Veilhan) discloses the use of a rigid oval dome over the wound site, its edges resting on the patient's skin. The climate within this dome is heated thus providing a warm wound environment. Other heated approaches are described in WO 98/31311 and WO 98/31310 (Augustine Medical) both of which utilise remote heating devices to provide an elevated temperature at the wound site. A simpler approach is described by inference in GB 2 285 729 (British Technology Group) where a heated device, in this case a heated cloth, could be directly applied to a wound surface.

The devices described above are all fairly complex and not compatible with wound care in a healthcare facility or the like. Furthermore, such devices are expensive and not fully proven to be effective in promoting good wound repair. Also, such devices purely address the wound site. Vascular dilation, the essence of heated wound repair , needs to take place where blood vessels enter and leave the wound site. The wound site needs to repair under optimum conditions and a whole range of mechanisms and materials are required for optimized treatment dependent on the wound types being considered. These include occlusive or semi-occlusive healing using materials ranging from water vapour permeable films through hydrocolloid or hydrogel structures to alginate derivatives. The presence of heat in synergy with these specialist dressing systems is unproven and hence possibly deleterious to wound repair.

Accordingly, this invention seeks to improve blood flow to wounds by vascular dilation of the vessels leading to and from the wound site by utilising materials well known for providing such properties. Such increased blood flow will improve wound healing as already described.

The essence of this invention is to use rubefacients which permeate through the epidermis and act to dilate blood vessels leading to and from the wound site. These act in a way simulating an elevated temperature. The enhanced blood flow will also remove catabolic products thus speeding up the wound healing process. A suitable rubefacient is applied by way of a carrier matrix which could advantageously be an adhesive dressing material applied to areas surrounding the wound. Such adhesive dressings are ideally of a doughnut shape with a hole to go over the wound area to avoid the presence there of rubefacient since the presence of rubefacient is extremely painful to the sufferer if this enters the wound area.

The choice of rubefacient is important in optimising dilation of the blood vessels leading to and from the woundsite. A well known rubefacient is methyl salicylate which is safe and well proven. A further advantage to this substance is its resistance without deterioration to steam autoclaving and other sterilisation techniques. Other appropriate rubefacients are described in Martindale. The Extra Pharmacopoeia, 30^{th} Edition, Pharmaceutical Press, London 1993. These can include but are not limited to capsaicin, Cayenne pepper, nonivamide or benzyl nicotinate.

Well known rubefacient plasters such as ABC-Warme-Pflaster (Beiersdorf GmbH) or Finalgon Schmerzpflaster (Thomae GmbH) are ideal materials from which to construct the invention described herein. A hole can be cut into the centre of such a plaster to allow the wound site to be open and rubefacient free. Furthermore, an appropriate wound dressing such as a hydrogel, hydrocolloid, film, alginate etc can be introduced or positioned into this free area if desired to provide for optimised wound healing. The rubefacient-containing plaster gives rise to effects similar to an elevated temperature due to blood vessel dilation to enhance wound healing.

It is important as already stressed to avoid rubefacient migration into the wound site. This can be achieved by the use of baffles to retain the rubefacient away from the wound site or to maintain a "free area" between the rubefacient and the wound site of sufficient size to prevent
rubefacient migration during dressing application or during its period of patient use. Incorporation of the rubefacient within the adhesive can secure its positioning. A version of this allows for the incorporation of rubefacient within an adhesive matrix by micro-encapsulation technology such that during dressing application, the rubefacient is released and hence can permeate the epidermal tissue and facilitate vascular dilation. A version of this approach allows for timed release of the rubefacient by using different microencapsulating polymers such that release of the rubefacient occurs over a timed basis, the microclimate at the interface of the microencapsule containing adhesive and the skin acting as a time trigger with some microcapsules breaking open before others.

Various embodiments of this invention are illustrated in the following diagrams. These are not exhaustive but show some of the preferred dressing formats.

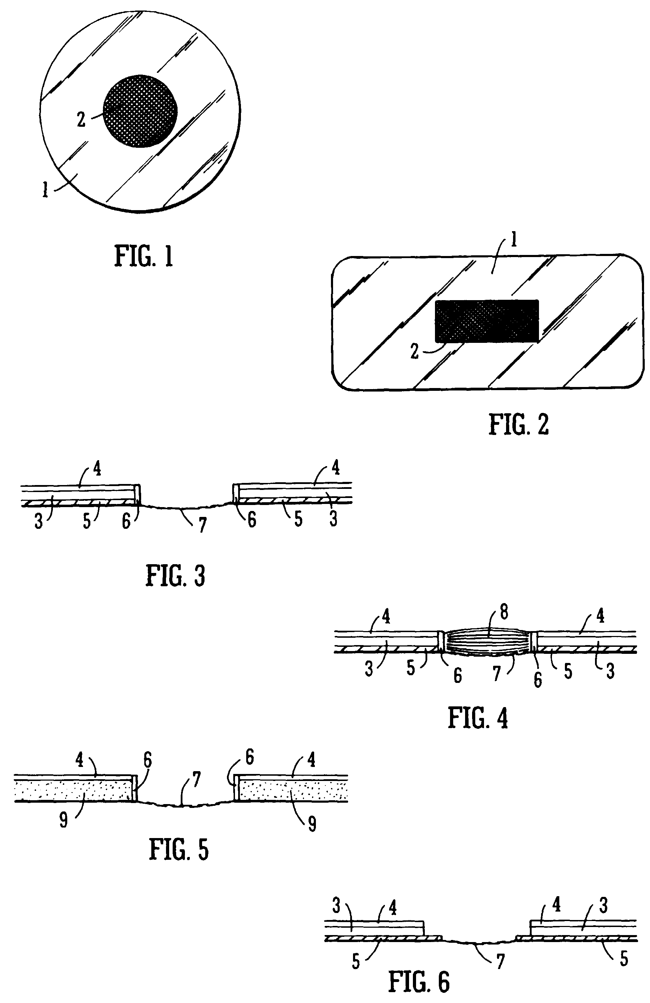

Figure 1 illustrates a general view of the top of a typical dressing according to the present invention. The dressing matrix (1) in this case is of round form and a round cut section (2) is larger in area than the wound area relevant to this dressing.
Figure 2 illustrates another top view of a typical dressing according to the present invention. In this case, the dressing matrix (1) is rectangular in shape as is the cut out section (2) since wounds are often rectangular in shape.
Figure 3 illustrates a cross-section of the dressings shown in Figures 1 and 2. In this example, the rubefacient layer (3) is disposed below a cover material (4) and above an adhesive layer (5). Baffles (6) made of a rubefacient impermeable material such as highly flexible and conformable plastic serve to prevent rubefacient entering the wound area (7).
Figure 4 illustrates a dressing as described in Figure 3 but with a wound dressing (of calcium alginate nonwoven in this case) (8) separately applied to the area over the woundsite.
Figure 5 illustrates a dressing as described in Figure 3 but with the rubefacient intermixed with adhesive as a single layer (9).
Figure 6 illustrates the deposition of rubefacient (3) in a typical embodiment of this invention situated away from the wound area (7).

## Claims

1. A wound dressing incorporating a rubefacient substance in such a disposition that, upon application of the dressing to a wound site, said substance will be brought into contact with skin surrounding the wound site but not with the wound site itself.

2. A wound dressing according to claim 1 comprising a layer of the rubefacient substance sandwiched between a cover material and an adhesive layer.

3. A wound dressing according to claim 1 comprising a cover material and a layer of adhesive, with the rubefacient substance incorporated into the adhesive layer.

4. A wound dressing according to claim 3 wherein the rubefacient substance is microencapsulated and dispersed through the adhesive layer.

5. A wound dressing according to any preceding claim formed with an aperture in a central region thereof.

6. A wound dressing according to any of claims 1 to 4 having a central area of rubefacient-free cover material.

7. A wound dressing according to any preceding claim including a barrier disposed to prevent the rubefacient substance spreading to a central region thereof.

8. A wound dressing according to any preceding claim wherein the rubefacient substance consists of or includes any one or more of methyl salicylate, capsaicin, Cayenne pepper, nonivamide, or benzyl nicotinate.

9. A wound dressing substantially as hereinbefore described with reference to and as illustrated by any of the accompanying drawings.
